(19) Europäisches Patentamt
European Patent Office
Office européen des brevets

(11) **EP 4 345 835 A1**

(12) **EUROPÄISCHE PATENTANMELDUNG**

(43) Veröffentlichungstag:
**03.04.2024   Patentblatt 2024/14**

(21) Anmeldenummer: **22198687.0**

(22) Anmeldetag: **29.09.2022**

(51) Internationale Patentklassifikation (IPC):
**G16H 30/40** (2018.01)   **G16H 30/20** (2018.01)
**A61B 5/05** (2021.01)   **A61B 6/00** (2024.01)
**G06N 3/08** (2023.01)   **G06N 3/02** (2006.01)

(52) Gemeinsame Patentklassifikation (CPC):
**G16H 30/20; A61B 6/54; G06N 3/084; G16H 30/40;**
A61B 6/03; A61B 6/481

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**
Benannte Validierungsstaaten:
**KH MA MD TN**

(71) Anmelder: **Siemens Healthineers AG**
**91301 Forchheim (DE)**

(72) Erfinder:
• **Taubmann, Oliver**
  **91365 Weilersbach (DE)**
• **Sühling, Michael**
  **91052 Erlangen (DE)**

(74) Vertreter: **Siemens Healthineers**
**Patent Attorneys**
**Siemens Healthcare GmbH**
**SHS TE IP**
**Henkestraße 127**
**91052 Erlangen (DE)**

(54) **VERFAHREN UND STEUEREINHEIT ZUR STEUERUNG EINER MEDIZINISCHEN BILDGEBUNGSANLAGE**

(57) Die Erfindung betrifft ein computerimplementiertes Verfahren zur Steuerung einer medizinischen Bildgebungsanlage (1), umfassend die Schritte
- Erfassen (S1) von patientenspezifischen Indikationsdaten mittels Schnittstelleneinheit,
- Erfassen (S2) wenigstens eines nutzer- und/oder einrichtungsspezifischen Präferenzparameters mittels Schnittstelleneinheit,
- automatisches Ermitteln (S3) eines Steuerdatensatzes basierend auf den Indikationsdaten und dem Präferenzparameter mittels Recheneinheit (21), und
- Steuern (S4) der medizinischen Bildgebungsanlage mit dem ermittelten Steuerdatensatz mittels Recheneinheit (21).

**FIG 1**

EP 4 345 835 A1

**Beschreibung**

Viele Schritte eines diagnostischen Bildgebungs- und Befundungsworkflows erfordern eine manuelle Interaktion des medizinischen Personals. Diese erfolgt über eine entsprechende Bedienschnittstelle an der medizinischen Bildgebungsanlage und/oder einer radiologischen Befundungsstation. Zur Durchführung der Bilddatenerfassung müssen bspw. Entscheidungen bezüglich des Scanprotokolls, der Rekonstruktionsparameter und der Nachbearbeitung der erzeugten Bilder getroffen und typischerweise manuell eingegeben werden. Mittels Automatisierung lässt sich dieser Prozess vereinfachen und rationalisieren. Die Anzahl erforderlicher Interaktionen lässt sich derart reduzieren. Dadurch wird Zeit gespart und ein Fehlerrisiko durch unerwünschte Variationen in den Workflows oder aufgrund inhärenter Standardisierung derselben reduziert. Bekannt sind auch automatisierte Nachbearbeitungslösungen für die erzeugten Bilddaten, um einen Radiologen beim Befunden zu unterstützen. Hierbei werden häufig viele verschiedene, standardisierte Nachbearbeitungsergebnisse automatisch erzeugt, was einer großen Verarbeitungsleistung und entsprechender Zeit sowohl bei der automatischen Erzeugung als auch der manuellen Sichtung, Auswahl und klinischen Bewertung durch den Radiologen bedarf.

Derlei Ansätze gewinnen durch das Aufkommen der photonenzählenden Detektortechnologie an Relevanz, denn das Detektieren der Röntgenstrahlung mittels photonenzählender Röntgendetektoren bringt per se eine höhere Datenmenge mit sich und bietet aufgrund der zusätzlich umfassten spektralen Information im Vergleich zur klassischen Röntgendetektortechnologie unzählige zusätzliche Optionen, um Ergebnisse in Form von (nachbereiteten) Bilddaten bereit zu stellen.

Eine Workflow-Automatisierung setzt die Kenntnis von patientenspezifischer Indikationsinformation in maschinell verarbeitbarer Form voraus. Anders ausgedrückt muss Kenntnis darüber bestehen, warum ein Patient einer medizinischen Bildgebung unterzogen werden soll, denn die Indikation entscheidet neben weiteren patientenbezogenen Faktoren, welche Untersuchung, also welches Bildgebungsprotokoll mit welchen Parametereinstellungen durchgeführt werden muss und welche Ergebnisse in Form von nachbearbeiteten medizinischen Bildern für den Patienten für eine sinnvolle Befundung benötigt werden.

Typischerweise wird die Indikationsinformation neben den weiteren Faktoren durch das medizinische Personal bei der Untersuchungsplanung manuell über eine entsprechende Eingabeeinheit angegeben oder sie liegen bereits in einem strukturierten, kodierten Format zur Weiterverwendung bereit, bspw. in einem Krankenhaus- oder Radiologie-Informationssystem (HIS/RIS - hospital/radiology information system) und/oder in einer elektronischen Patientenakte (EHR/EMR-electronic health/medical record). Alternativ kann die Information in unstrukturierter Form vorhanden sein, bspw. in Form einer Überweisung, früherer Bilddaten und/oder eines früheren Befundberichts des Patienten. Mittels an sich bekannter Methoden zur Verarbeitung natürlicher Sprache (NLP - natural language processing) oder Methoden der Bildanalyse kann auch hier die Indikationsinformation und ggf. weitere Faktoren extrahiert werden.

Einen Vorschlag zur Nutzbarmachung von vorhandener Indikationsinformation zur automatischen Ableitung von Steuerdatensätzen gibt die Europäische Patentanmeldung EP 3451211 A1 an. Hier werden Indikationsdaten unter Anwendung einer Ontologie in entsprechende Steuerdatensätze zur Durchführung von Erfassungs-, Rekonstruktions- und/oder Nachbereitungsschritten überführt, wodurch auf manuelle Eingaben weitgehend verzichtet werden kann.

In der Praxis zeigt sich jedoch, dass es häufig nachträglich zu manuellen Anpassungen der automatisiert bereitgestellten Steuerdatensätze kommt, wobei die Anpassungen spezifisch für einen bestimmten Nutzer und/oder eine bestimmte medizinische Einrichtung bzw. die Region der medizinischen Einrichtung sind.

Bekannt sind Lösungen, die einzelne Nachbearbeitungsschritte für die erzeugten Bilder an das ausgewählte Bildgebungsprotokoll binden, diese Verknüpfung ist jedoch weder patienten noch nutzerspezifisch.

Ausgehend davon ist es Aufgabe der vorliegenden Erfindung, Mittel bereit zu stellen, die von vornherein nicht nur eine patienten-, sondern auch eine nutzer- und/oder eine einrichtungsspezifische Automatisierung eines medizinischen Bildgebungsworkflows erlauben. Insbesondere ist es Aufgabe der vorliegenden Erfindung, den Bedarf für eine manuelle Interaktion zwischen Nutzer und medizinischer Bildgebungsanlage weiter zu reduzieren.

Diese Aufgabe wird gelöst durch ein Verfahren zur Steuerung einer medizinischen Bildgebungsanlage, eine entsprechende Steuereinheit, eine medizinische Bildgebungsanlage umfassend die Steuereinheit sowie ein entsprechendes Computerprogrammprodukt und ein computerlesbares Medium gemäß den unabhängigen Ansprüchen. Bevorzugte und/oder alternative, vorteilhafte Ausgestaltungsvarianten sind Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind ebenso auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer der Vorrichtungen beschrieben oder beansprucht sind, weitergebildet sein. Die entsprechenden funktionalen Merkmale des Verfahrens werden dabei durch entsprechende gegenständliche Module oder Einheiten ausgebildet.

Ein erster Aspekt ist auf ein Verfahren zur Steuerung einer medizinischen Bildgebungsanlage gerichtet. Das Verfahren ist als computerimplementiertes Verfahren ausgebildet, das heißt, dass zumindest einzelne oder auch alle Schritte des Verfahren von einem Computer bzw. einem Rechenmodul bzw. einer Steuereinheit ausgeführt werden.

Entsprechend betrifft ein weiterer Aspekt der vorliegenden Erfindung eine Steuereinheit zur Steuerung einer medizinischen Bildgebungsanlage. Die Steuereinheit ist in Form oder als Bestandteil einer Datenverarbeitungseinheit ausgebildet und eingerichtet und umfasst Mittel zur Durchführung einzelner oder aller Schritte des erfindungsgemäßen Verfahrens.

Die Steuereinheit umfasst

- eine Schnittstelleneinheit, die ausgebildet ist zum

  - Erfassen von patientenspezifischen Indikationsdaten, und
  - Erfassen wenigstens eines nutzer- und/oder einrichtungsspezifischen Präferenzparameters mittels Schnittstelleneinheit,

- eine Recheneinheit, die ausgebildet ist zum

  - automatischen Ermitteln eines Steuerdatensatzes basierend auf den Indikationsdaten und dem Präferenzparameter, und
  - Steuern der medizinischen Bildgebungsanlage mit dem ermittelten Steuerdatensatz.

Das erfindungsgemäße Verfahren umfasst eine Vielzahl von entsprechenden Schritten.

Ein erster Schritt umfasst das Erfassen von patientenspezifischen Indikationsdaten mittels Schnittstelleneinheit.

Ein weiterer Schritt umfasst das Erfassen wenigstens eines nutzer- und/oder einrichtungsspezifischen Präferenzparameters mittels Schnittstelleneinheit.

Ein weiterer Schritt umfasst das automatische Ermitteln eines Steuerdatensatzes basierend auf den Indikationsdaten und dem Präferenzparameter mittels Recheneinheit.

Ein weiterer Schritt umfasst das Steuern der medizinischen Bildgebungsanlage mit dem ermittelten Steuerdatensatz mittels Recheneinheit.

Eine medizinische Bildgebungsanlage umfassend eine oben beschriebene Steuereinheit bildet einen dritten Aspekt der Erfindung. Eine medizinische Bildgebungsanlage ist eine Bildgebungsanlage zum Einsatz in der Medizin. Eine erfindungsgemäße medizinische Bildgebungsanlage verwendet zur Bilderzeugung bspw. Röntgenstrahlung und umfasst bspw. jeweils wenigstens einen Röntgenstrahler sowie einen Röntgenstrahlendetektor. Alternativ kann die medizinische Bildgebungsanlage Magnetfelder für die Bilddatenerfassung einsetzen.

Insofern kann die medizinische Bildgebungsanlage in Ausführungen in Form eines C-Bogengeräts, eines Computer-Tomographen oder eines Radiographie-Geräts oder einer Magnetresonanztomographie-Anlage ausgebildet sein. In weiteren Ausführungen kann die medizinische Bildgebungsanlage auch andere Bilderzeugungstechniken anwenden, bspw. Ultraschall, Positronen-Emissions-Tomographie oder andere.

Die medizinische Bildgebungsanlage umfasst in Ausführungen neben einer Einheit zur Bilddatenerzeugung auch eine Einheit zur Betrachtung, visuellen Begutachtung bzw. der Befundung der erzeugten Bilddaten. Mit anderen Worten stellt die medizinische Bildgebungsanlage bspw. einen Radiologie-Arbeitsplatz bereit. Der Radiologie-Arbeitsplatz kann dabei direkt neben der Einheit zur Bilddatenerzeugung in einem Untersuchungsraum, in einem benachbarten Steuerraum und/oder abgelegen von der Einheit zur Bilddatenerzeugung angeordnet sein, bspw. in einer Radiologie-Abteilung derselben medizinischen Einrichtung, einem räumlich von der medizinischen Einrichtung getrennten, eigenständigen Radiologie-Zentrum. Sowohl die Einheit zur Bilddatenerzeugung als auch der Radiologie-Arbeitsplatz umfassen jeweils Bedieneinheiten für einen Nutzer, über welche eine Mensch-Maschine-Interaktion realisiert wird. Die Bedieneinheiten umfassen vorteilhaft jeweils eine von der Steuereinheit umfasste Schnittstelleneinheit. Eine Schnittstelleneinheit umfasst dabei eine Ausgabeschnittstelle, bspw. in Form einem Monitors. Insbesondere werden darüber erzeugte Bilddaten angezeigt. Die Schnittstelleneinheit umfasst ferner eine Eingabeeinheit zum Erfassen eines Präferenzparameters und/oder weiterer Eingabedaten für eine Weiterverarbeitung.

In Ausführungen ist die Steuereinheit weiter über entsprechende Datenschnittstellen, die auch von der Schnittstelleneinheit umfasst sein können, mit der medizinischen Bildgebungsanlage, insbesondere den bildgebenden Komponenten wie Röntgenstrahler oder Röntgenstrahlendetektor zur Datenkommunikation verbunden. Hierüber kann Steuerinformation und/oder erfasste Bildinformation zur Weiterverarbeitung übertragen werden. In Ausführungen ist die Steuereinheit in die medizinische Bildgebungsanlage integriert. Alternativ kann die Steuereinheit auch entfernt bzw. abgelegen von den bildgebenden Komponenten, aber auch von dem Radiologie-Arbeitsplatz angeordnet sein. Die Steuereinheit ist ferner ausgebildet, die Schritte des automatischen Ermittelns eines Steuerdatensatzes basierend auf den Indikationsdaten und dem Präferenzparameter sowie des Steuerns der medizinischen Bildgebungsanlage mit dem ermittelten Steuerdatensatz auszuführen. Dazu umfasst sie wenigstens eine Recheneinheit.

Die Steuereinheit kann als zentrale oder dezentrale Rechen- oder Datenverarbeitungseinheit bzw. Computer ausgebildet sein. Die Steuereinheit kann einen oder mehrere Prozessoren aufweisen. Die Prozessoren können als zentrale Verarbeitungseinheit (central processing unit, CPU) und/oder als Grafikprozessor (graphics processing unit, GPU) ausgebildet sein. Alternativ kann die Steuereinheit als lokaler oder Cloud-basierter Verarbeitungsserver implementiert sein.

Die Schnittstelleneinheit kann allgemein auch zum Datenaustausch zwischen der Steuereinheit und weiteren Komponenten der medizinischen Bildgebungsanlage ausgebildet sein. Die Schnittstelleneinheit kann auch zum Datenaustausch zwischen Komponenten der Steuereinheit ausgebildet sein. Die Steuereinheit kann in Form von einer oder mehreren einzelnen Schnittstellen implementiert sein, welche beispielsweise ein Hardware- und/oder Software-Interface, z.B. einen PCI-Bus, eine USB-Schnittstelle, eine Fire-Wire-Schnittstelle, eine Zig-Bee- oder eine Bluetooth-Schnittstelle aufweisen können. Die Schnittstelleneinheit kann ferner eine Schnittstelle eines Kommunikationsnetzwerks aufweisen, wobei das Kommunikationsnetzwerk ein Local Area Network (LAN), beispielsweise ein Intranet oder ein Wide Area Network (WAN) aufweisen kann. Entsprechend kann die Schnittstelleneinheit eine LAN-Schnittstelle oder eine Wireless LAN-Schnittstelle (WLAN oder Wi-Fi) aufweisen.

In Ausführungen der Erfindung kann die Steuereinheit bspw. einen Datenspeicher aufweisen, der über die Schnittstelleneinheit mit der Recheneinheit in Datenverbindung steht. In dem Datenspeicher können bspw. neben weiteren patientenspezifischen Daten Indikationsdaten zu einem Patienten abrufbar für die Recheneinheit hinterlegt sein. In dem Datenspeicher können alternativ oder zusätzlich auch Listen umfassend Werte für Bildgebungs-, Rekonstruktions- und/oder Nachbearbeitungsparameter abrufbar hinterlegt sein. Der Datenspeicher kann vorab definierte Zuordnungen und/oder Zusammenhänge zwischen in den Indikationsdaten umfassten Indikationsparametern und Werten für Bildgebungs-, Rekonstruktions- und/oder Nachbearbeitungsparameter umfassen. Der Datenspeicher kann als lokaler Speicher der medizinischen Bildgebungsanlage, z.B. als Arbeitsspeicher oder Festplatte, oder als dezentraler oder zentraler Speicher, bspw. einem EHS-System, einem Krankenhausinformationssystem (HIS) oder einem Radiologieinformationssystem (RIS) einer medizinischen Einrichtung, bspw. einem Krankenhauses, ausgebildet sein. Weiterhin kann der Datenspeicher Teil eines Serversystems sein.

Schritt eins des Verfahrens betrifft das Erfassen von patientenspezifischen Indikationsdaten. Indikationsdaten sind dabei als Daten über eine medizinische Indikation zu verstehen, welche einen Patienten betreffen, bzw. ein Krankheitsbild, welches ein Nutzer/Befunder bei diesem Patienten für wahrscheinlich erachtet. Die Indikationsdaten können auch Angaben oder Information über eine vorzunehmende Prozedur, insbesondere eine medizinische Bildgebungsprozedur, beinhalten. Die Indikationsdaten können in strukturierter, aber auch unstrukturierter Form vorliegen. Die Indikationsdaten können in Ausführungen einer klinischen Konstellation aus einer Vielzahl von an sich bekannten klinischen Konstellationen entsprechen. Eine klinische Konstellation bezeichnet dabei einen durch die Indikationsdaten repräsentierten medizinischen bzw. anatomischen Gesamtzustand des Patienten. Die Schnittstelleneinheit kann zum Erfassen der Indikationsinformation z.B. mit einem HIS oder einem RIS in Datenkommunikation stehen. Die Indikationsdaten können in Ausführungen auch von einer Modality Worklist der medizinischen Bildgebungsanlage über die Schnittstelleneinheit abgerufen werden. Die Modality Worklist bildet die Indikationsdaten, die bspw. in einer RIS-Terminologie vorliegen, direkt auf DICOM Attribute ab (DICOM = Digital Imaging and Communications in Medicine).

Die Indikationsdaten repräsentieren also Vorwissen über den Patienten. Die Indikationsdaten können in einer Ausführung in freier Textform vorliegen. In anderer Ausführung können die Indikationsdaten in einem Audioformat, bspw. in Form eines diktierten Befundberichts zu dem Patienten entsprechen. In diesen Fällen kann das Erfassen der Indikationsdaten auch eine semantische Analyse oder andere Verfahren zur Verarbeitung von natürlicher Sprache (NLP) umfassen, um die freitextlich gestalteten Indikationsdaten für die Recheneinheit zugänglich und maschinell weiterverarbeitbar zu machen. Die Indikationsdaten können auch von früheren medizinischen Bilddaten des zu untersuchenden Patienten umfasst sein. Das Erfassen der Indikationsdaten umfasst in Ausführungen entsprechend eine Extraktion eines Befundes bzw. eines Befunds für den Patienten mittels bekannter Bildanalyseverfahren.

Die Indikationsdaten umfassen in Ausführungen der Erfindung einen oder eine Mehrzahl von Indikationsparametern, die zusammen betrachtet eine Indikation für den Patienten repräsentieren.

Schritt zwei betrifft das Erfassen wenigstens eines nutzer- und/oder einrichtungsspezifischen Präferenzparameters. Dieser Schritt umfasst folglich eine Interaktion zwischen Nutzer und Steuereinheit über die Schnittstelleneinheit. Der Nutzer kann nun über eine haptische, akustische und/oder gestenbasierte Interaktion mit der medizinischen Bildgebungsanlage einen Präferenzparameter eingeben. Der Präferenzparameter ist dabei spezifisch für die erfassten Indikationsdaten zu dem zu untersuchenden Patienten. Der Präferenzparameter kennzeichnet dabei in Ausführungen eine bevorzugte Einstellung bzw. einen präferierten Wert für einen Parameter eines medizinischen Bildgebungsprotokolls bzw. für ein Bildrekonstruktions- oder Bildnachbearbeitungsverfahren. Kennzeichnend für den Präferenzparameter ist erfindungsgemäß, dass dieser nutzer- und/oder einrichtungsspezifisch ist. In der Praxis bevorzugen verschiedene Nutzer zu vergleichbaren oder identischen Indikationsdaten verschiedene Bildgebungsprotokolle bzw. verschiedene Werte für einzelne oder mehrere Protokollparameter bzw. verschiedene Vorgaben für die Bildrekonstruktion bzw. die nachträgliche Bildbearbeitung für die Befundung. Ferner lassen sich lokale oder regionale, also einrichtungsspezifische Unterschiede im Hinblick auf die Handhabung einer Behandlung/Untersuchung von Patienten mit gleichem Befund oder ähnlicher Indikation bzw. ähnlicher medizinischer Konstellation von der Bildgebung bis zur Befundung beobachten. So bestehen bspw. regionale Unterschiede in der Gestaltung von Standard-Bildaufnahme/Bildgebungs-Protokollen zu ein und derselben Indikation. Das führt dazu, dass in bestimmten Einrichtungen anderen Ortes als etabliert geltende Bildgebungsprotokolle nicht verfügbar sind und umgekehrt. Das erfindungsgemäße Erfassen des nutzer- und/oder einrichtungsspezifischen Präferenzparameters ermöglicht vorteilhaft eine Berücksichtigung dieser Präferenzen/Unterschiede bei der Ermittlung des zu den erfassten Indikationsdaten gehörigen Steuerdatensatzes. Die Erfindung geht davon aus, dass über die Nutzereingabe sowohl ein nutzer- und/oder ein einrichtungsspezifischer Präferenzparameter erfasst wird. Ein Nutzer gibt folglich in Ausführungen auch Präferenzparameter vor, die spezifisch für die medizinische Einrichtung sind. In Ausführungen der Erfindung wird nicht nur ein Präferenzparameter, sondern eine Vielzahl von Präferenzparametern, bspw. zwei, drei, vier oder mehr, erfasst.

Der wenigstens eine Präferenzparameter entspricht in Ausführungen der Erfindung einem Parameter für die Bilddatenerfassung mittels der medizinischen Bildgebungsanlage und/oder für eine Bildrekonstruktion aus den erfassten Bilddaten und/oder für eine Nachbearbeitung der rekonstruierten Bilddaten. Der Präferenzparameter entspricht in Ausführungen also einer Wertevorgabe für einen Protokollparameter eines Bildgebungsprotokolls, eines Rekonstruktionsprotokolls oder eines Nachbearbeitungsprotokolls. Der Präferenzparameter ist in Ausführungen der Erfindung ein Parameter aus der Gruppe der folgenden Parameter: Field of View, Kontrastmittel, Matrixgröße, Dosis, Rekonstruktionskern, Filterung, virtuell native Rekonstruktion, Energieniveau für monoenergetische Bilder, Jodkarte, Rendering Parameter, spezielle Segmentierung.

Schritt drei des Verfahrens betrifft das automatische Ermitteln eines Steuerdatensatzes basierend auf den Indikationsdaten und dem Präferenzparameter. Das automatische Ermitteln umfasst ein maschinelles, indikationsspezifisches Zuordnen von Steuerdaten zu den Indikationsdaten. Der ermittelte Steuerdatensatz umfasst in Ausführungen eine (vollständige) Menge von Wertevorgaben für die Protokollparameter eines Bildaufnahme-Protokolls. Der ermittelte Steuerdatensatz umfasst in weiteren Ausführungen Wertevorgaben für Rekonstruktionsparameter eines Bildrekonstruktionsalgorithmus. In weiteren Ausführungen umfasst er Wertevorgaben für einen Bild-(nach-)bearbeitungsalgorithmus zur Anwendung auf die rekonstruierten Bilddaten. Allgemein ist im Verlauf der Anmeldung in Bezug auf die einzelnen vom Steuerdatensatz umfassten Parameter von Protokollparametern die Rede. Insofern umfasst der Steuerdatensatz in Ausführungen des Verfahrens wenigstens einen Steuerbefehl in Bezug auf eine Bilddatenerfassung mittels der medizinischen Bildgebungsanlage und/oder in Bezug auf eine Bildrekonstruktion aus den erfassten Bilddaten und/oder in Bezug auf eine Nachbearbeitung der rekonstruierten Bilddaten umfasst.

Das automatische Ermitteln dieser Wertevorgaben berücksichtigt erfindungsgemäß nicht mehr nur die Indikationsdaten, sondern auch den wenigstens einen Präferenzparameter, sodass in Schritt drei eine von einer Standard-Zuordnung zwischen Indikationsdaten und Steuerdaten abweichende Zuordnungsvorschrift angewandt wird.

Damit ermöglicht die Erfindung vorteilhaft das automatisierte Modifizieren von standardisierten Bilderfassungs-, Rekonstruktions- und/oder Bearbeitungsprotokollen und damit das Erzeugen von neuen, bislang nicht verfügbaren Bilderfassungs-, Rekonstruktionsund/oder Bearbeitungsmethoden.

Schritt vier betrifft das Steuern der medizinischen Bildgebungsanlage mit dem ermittelten Steuerdatensatz, also die Ausführung der Steuerdaten. Der ermittelte Steuerdatensatz wird folglich von der Recheneinheit über die Schnittstelleneinheit an wenigstens eine, bevorzugt mehrere Komponenten der medizinischen Bildgebungsanlage übertragen und dort entsprechend der umfassten Wertvorgaben für die Protokollparameter zur Ausführung gebracht. Anhand der Wertevorgaben des ermittelten Steuerdatensatzes werden folglich Bilddaten erfasst, rekonstruiert und/oder nachbearbeitet.

In Ausführungen der Erfindung umfasst das automatische Ermitteln, automatisch einen Steuerdatensatz entsprechend den patientenspezifischen Indikationsdaten aus einer vorab definierten Zuordnung zwischen einer Vielzahl von verschiedenen Indikationsdaten und einer Vielzahl von verschiedenen Steuerdatensätzen auszuwählen. Diese Vorgehensweise berücksichtigt zunächst nicht den oder die erfassten Präferenzparameter, sondern nutzt an sich bekannte, übliche bzw. vorab definierte Zuordnungen zwischen Indikationsdaten und den Steuerdaten. Eine bekannte Zuordnung kann bspw. wie in der Europäischen Patentanmeldung EP 3451211 A1 beschrieben, unter Anwendung einer Ontologie bzw. einer Steuerdatenbibliothek ausgebildet sein. Der Steuerdatensatz wird dort aus einer Steuerdaten-Bibliothek in Abhängigkeit von den in die Ontologie abgebildeten Indikationsdaten ermittelt. Diese Ermittlung geschieht mittels Inferenz (logische Schlussfolgerungen) und/oder numerischer Modellierung. Mittels Inferenz werden Terme der Ontologie (Indikations-Ontologie) und der Steuerdaten-Bibliothek zueinander in Beziehungen gesetzt und logische Schlussfolgerungen abgeleitet, um den geeigneten Steuerdatensatz zu ermitteln. Bei der numerischen Modellierung geschieht die Ermittlung auf numerischem Wege. Numerische Modelle geben für gegebene Indikationsdaten numerische Wahrscheinlichkeiten für Steuerdatensätze aus. Alternativ dazu kann bspw. der oben eingeführte Datenspeicher eine Datenbank-ähnliche, vorab festgelegte Zuordnungsvorschrift zwischen Indikationsparametern der Indikationsdaten und von den Steuerdaten umfassten Steuerparametern umfassend entsprechende Protokollparameter zur Bilddatenerfassung, zur Rekonstruktion und/oder zur Bildnachbearbeitung umfassen. In Ausführungen kann die vorab festgelegte Zuordnungsvorschrift auf einer manuellen Festlegung durch einen Nutzer und/oder einen Administrator beruhen. Die Recheneinheit vergleicht nun im Rahmen des automatischen Ermittelns in Ausführungen die erfassten Indikationsdaten parameterweise mit den Indikationsparametern in der Datenbank und ordnet die zugehörigen Protokollparameterwerte zu.

Insbesondere entsprechen diese an sich bekannten Zuordnungsvorschriften allgemein anwendbaren medizinischen Leitlinien (medical guidelines), die zu jeder Zeit einer Behandlung/Untersuchung eines Patienten strikt eingehalten werden müssen.

In dieser erfindungsgemäßen Verfahrensvariante werden den Indikationsdaten zunächst Standard-Steuerdatensätze umfassend Protokollparameter zu etablierten und weit verbreitet angewandten Bilddatenerfassungs-, Rekonstruktions-, und Nachbearbeitungsprotokollen zugeordnet.

In Weiterbildung dieser Ausführung umfasst das automatische Ermitteln in einem Folgeschritt, die automatische Auswahl eines Steuerdatensatzes basierend auf dem wenigstens einen Präferenzparameter anzupassen. Mit anderen Worten wird in Weiterbildung eine nutzer- oder einrichtungsseitige Präferenz zur Gestaltung eines Bilddatenerfassungs-, Rekonstruktions-, und Nachbearbeitungsprotokolls nachträglich berücksichtigt, indem wenigstens einer der vorab zugeordneten Protokollparameter entsprechend eines Standard-Protokolls an den erfassten Präferenzparameter angepasst wird.

Diese Vorgehensweise ermöglicht vorteilhaft, zunächst einer erfassten Indikation an sich bekannte und weit verbreitete Standard-Protokolle zuzuordnen und dabei auf ebenfalls an sich bekannte automatische oder manuelle Verfahren für die Zuordnung zurückzugreifen.

In weiterer Ausführung des erfindungsgemäßen Verfahrens umfasst das automatische Ermitteln des Steuerdatensatzes, eine trainierte Funktion des maschinellen Lernens auf die Indikationsdaten anzuwenden. Mit anderen Worten wird aus den die Indikationsparameter umfassenden Indikationsdaten ein Steuerdatensatz automatisch abgeleitet, wobei die Indikationsdaten als Eingabewerte einer trainierten Funktion bzw. eines trainierten Algorithmus des Maschinen-Lernens verwendet werden.

Maschinelles Lernen im Sinne der Erfindung umfasst eine computer-implementierte Technik, bei der ein Algorithmus auf Basis von bestehenden Daten Muster bzw. Gesetzmäßigkeiten erkennt und unter Anwendung derselben in Bezug auf unbekannte, neue Daten eigenständig Lösungen ableitet. Voraussetzung für eine eigenständige Lösungsfindung ist eine Trainingsphase, in der ein Algorithmus des Maschinen-Lernens auf einen bekannten, definierten und zumeist sehr großen Datenbestand angewandt wird, um diejenigen Regeln bzw. Vorhersagen zu finden, die eine gewünschte Ausgabe bzw. ein gewünschtes Ergebnis erzielen. Das Training kann als überwachtes oder unüberwachtes Training ausgebildet sein, wobei in der ersten Variante dem Algorithmus Werte-Paare in Form von Eingabewerten und dazu gehörigen, korrekten Ausgabewerten präsentiert werden, wohingegen in der zweiten Variante der Algorithmus sich basierend auf den Eingabewerten eigenständig derart selber anpassen muss, dass er die korrekten Ausgabewerte liefert.

Besonders vorteilhaft ist die Funktion des Maschinen-Lernens als künstliches neuronales Netz ausgebildet. Ein künstliches neuronales Netz orientiert sich am Aufbau eines biologischen, neuronalen Netzes wie bspw. einem menschlichen Gehirn. Ein künstliches neuronales Netz umfasst zwischen einer Eingabe- und einer Ausgabeschicht bevorzugt eine Vielzahl von weiteren Schichten jeweils umfassend wenigstens einen Knoten. Jeder Knoten entspricht dabei einer Verarbeitungseinheit, analog einem biologischen Neuron. Knoten innerhalb einer Schicht des Netzes können über gerichtete Verbindungen (edges) mit Knoten anderer Schichten verbunden sein. Die Verbindungen definieren den Datenfluss innerhalb des Netzes. Jeder Knoten repräsentiert folglich eine Operation, die auf die Eingabedaten angewendet wird. Ferner verfügt jeder Knoten bzw. jede seiner Verbindungen über einen Gewichtungsparameter (weight). Über diesen Gewichtungsparameter wird der Einfluss bzw. die Wichtigkeit der Ausgabe eines Knotens als Eingabewert für einen Empfängerknoten definiert. In der Trainingsphase, die bevorzugt als überwachtes Lernen ausgeführt wird, 'lernt' das künstliche Neuronale Netz anhand der Trainingsdaten die Gewichtungsparameter für alle Knoten bzw. Verbindungen und passt diese so lange an, bis die Ausgabeschicht des Netzes die korrekten Ausgabewerte liefert.

Besonders bevorzugt kommt ein Algorithmus des sogenannten Deep Learnings, bspw. in Form eines , convolutional neuronal networks', auch faltendes neuronales Netz, zum Einsatz. Die trainierte Funktion ist also insbesondere ausgebildet, eine Klassifikation durchzuführen, wobei der Menge der eingegebenen Indikationsparameter und ggf. dem eingegebenen Präferenzparameter eine definierte Protokollparametermenge umfassend wenigstens einen (Wert für einen) Protokollparameter für eine Bilddatenerfassung, Bildrekonstruktion und/oder Bildnachbearbeitung zugeordnet wird. Alternativ zu einem faltenden neuronalen Netz können auch Lange Kurzzeit-Gedächtnis (LSTM long short-term memory) Netze oder rekurrente neuronale Netze (RNN) zum Einsatz kommen, welche im Gegensatz zu den vorher genannten rückwärts gerichtete Feedback-Schleifen innerhalb der versteckten Netzschichten aufweisen.

Verfahrensgemäß kann die trainierte Funktion des Maschinen-Lernens ausgebildet sein, zunächst eine Standard-Zuordnung von Steuerdaten zu den erfassten Indikationsdaten vornehmen, entsprechend von anerkannten, etablierten Protokolle der Bildgebung, Rekonstruktion und/oder Bildnachbearbeitung. Die trainierte Funktion kann ferner ausgebildet sein, erst in einem nächsten Teilschritt den wenigstens einen Präferenzparameter zu berücksichtigen und die Standard-Steuerdaten entsprechend dem Präferenzparameter zu modifizieren. Im Detail kann die trainierte Funktion wenigstens eine basierend auf der Standard-Zuordnung festgelegte Wertevorgabe für die Menge der Protokollparameter an den Präferenzparameter anpassen.

Alternativ dazu kann die trainierte Funktion ausgebildet sein, direkt die nutzer- und/oder einrichtungsspezifische Protokollparametermenge für den Steuerdatensatz zu bestimmen. Diese erfindungsgemäße Ausführung ist gegenüber vorher beschriebenen Verfahrensvarianten geeignet, besonders schnell einen Steuerparameter zu ermitteln und den medizinischen Ablauf dadurch deutlich zu beschleunigen.

In Ausführungen der Erfindung dient also auch der wenigstens eine erfasste Präferenzparameter als Eingabewert für die trainierte Funktion des Maschinen-Lernens. Hier ordnet die trainierte Funktion des maschinelles Lernens den Indikationsparametern und dem Präferenzparameter gemeinsam einen passenden Steuerdatensatz umfassend eine Protokollparametermenge zu. In wieder anderen Ausführungen wird der Präferenzparameter nicht als Eingabewert für die trainierte Funktion verwendet, sondern fungiert als Stellglied zur Festlegung/Anpassung der Gewichte der einzelnen Knoten des Netzes im Rahmen eines Lernprozesses des Netzes.

In Ausführung der Erfindung ist die trainierte Funktion des Maschinen-Lernens eine für einen Nutzer spezifische Funktion bzw. umfasst für verschiedene Nutzer spezifische Programmabschnitte. In dieser Ausführung kann vorgesehen sein, dass eine Nutzerkennung zur Identifikation verschiedener Nutzer als Eingabewert an die trainierte Funktion übergeben wird, um den Nutzerspezifischen Programmteil zu aktivieren. In alternativer Ausführung agiert die trainierte Funktion des maschinellen Lernens basierend auf den Indikationsdaten und dem Präferenzparameter als Eingabedaten unabhängig vom jeweiligen Nutzer.

Die erfindungsgemäße Vorgehensweise beruht auf der Erkenntnis, dass eine trainierte Funktion des Maschinen-Lernens im Rahmen ihres Trainings einen festen Zusammenhang zwischen Eingabewerten, hier in Form von für einen Patienten spezifischen Indikationsparametern und ggf. dem Präferenzparameter bzw. einer Nutzerkennung, und Ausgabewerten in Form von entsprechenden Protokollparametersätzen für den Steuerdatensatz, herstellt. Insofern ermöglicht die Erfindung vorteilhaft eine automatische und individualisierte Ermittlung eines Satzes von Protokollparametern für eine Bilddatenerfassung, Bildrekonstruktion und/oder Bildnachbearbeitung und die Erzeugung eines entsprechenden Steuerdatensatzes.

Die trainierte Funktion ist in bevorzugter Ausführung als eine Funktion ausgebildet, die mit Trainingsdaten umfassend Trainingseingabedaten in Form einer Vielzahl von Indikationsdaten und einer Vielzahl von Präferenzparametern und Trainingsausgabedaten in Form einer Vielzahl von entsprechenden Steuerdatensätzen trainiert wurde. In Ausführungen der Erfindung umfassen die Trainingseingabedaten auch eine Nutzerkennung. Derart kann die trainierte Funktion von vornherein einen für einen Nutzer oder eine Einrichtung spezifischen Zusammenhang zwischen Indikationsdaten und Steuerdatensatz für Bilddatenerfassung, Rekonstruktion und/oder Bildnachbearbeitung erlernen.

Insofern sei hier angemerkt, dass das Erfassen von Indikationsdaten und das Erfassen wenigstens eines, bevorzugt einer Vielzahl von Präferenzparametern nicht zeitlich korrelieren muss. Insbesondere ist erfindungsgemäß vorgesehen, dass der wenigstens eine erfasste Präferenzparameter aus bereits abgeschlossenen Untersuchungen von Patienten als Teilmenge von Trainingseingabedaten verwendet wird. In Ausführungen der Erfindung erfolgt das Erfassen des Präferenzparameters also (lange) vor einer Ermittlung eines Steuerdatensatzes. In anderen Ausführungen erfolgt das Erfassen des Präferenzparameters erst im Sinne einer Korrekturschleife erst nachdem eine Ermittlung eines Steuerdatensatzes erfolgt ist. Dies wird weiter unten noch genauer beschrieben.

Gemäß einer weiteren Ausführung des erfindungsgemäßen Verfahrens ist das Trainieren des Algorithmus des Maschinen-Lernens als eingangs schon erläutertes, überwachtes Lernen ausgebildet und erfolgt basierend auf einer Vielzahl von Paaren aus Indikationsparametern und einem jeweils zugeordneten Steuerdatensatz jeweils umfassend einen Satz an Protokollparametern. Unter Einbeziehung wenigstens eines Präferenzparameters als Teil der Trainingseingabedaten wird die erfindungsgemäß Zuordnung spezifisch für Nutzerpräferenzen oder spezifisch für eine Einrichtung bzw. eine Region. Zum Trainieren der Funktion des maschinellen Lernens wird üblicherweise die Methode der Fehlerrückführung (backpropagation) angewandt, welche darin besteht, eine Abweichung zwischen tatsächlichem und Soll-Ausgabewert, der aus den Trainingsdaten bekannt ist, dem künstlichen neuronalen Netz zur Anpassung der individuellen Gewichte von Knoten zurückzuspielen. Die jeweils zum Einsatz kommenden Trainingsdaten beruhen bevorzugt auf einer Vielzahl von tatsächlich erfolgten Untersuchungen von Patienten oder werden alternativ zumindest teilweise künstlich durch Simulation erzeugt.

Besonders flexibel lässt sich das erfindungsgemäße Verfahren einsetzen, wenn die trainierte Funktion basierend auf abgeschlossenen automatischen Ermittlungen eines Steuerdatensatzes kontinuierlich aktualisiert wird. Mit anderen Worten ist die trainierte Funktion als ein System des kontinuierlichen Lernens (CL - Continuous Learning) ausgebildet. In dieser Ausführung ist die trainierte Funktion in der Lage, einen initialen Lernprozess mittels der Trainingseingabedaten und der Trainingsausgabedaten und damit das Modell für die Zuordnung von Steuerparametern in Form von Protokollparametern für die Bilddatenerfassung, Rekonstruktion und Nachbearbeitung kontinuierlich zu aktualisieren. Dazu werden erfindungsgemäß kontinuierlich die erfassten Präferenzparameter jüngst ausgeführter Untersuchungen überwacht und abgespeichert. Bspw. werden die erfassten Präferenzparameter im oben eingeführten Datenspeicher als Menge von zweiten Trainingseingabedaten zu zweiten Trainingsausgabedaten in Form der zugeordneten Steuerdaten abgespeichert. In vorab festgelegten Intervallen, bspw. nach einer, zwei, drei oder mehr abgeschlossenen Untersuchungen, durchläuft die trainierte Funktion einen Aktualisierungslernprozess unter Anwendung der zweiten Trainingseingabedaten und Trainingsausgabedaten und passt die Gewichte einzelner Knoten des Netzes an die zweiten Trainingseingabedaten und zweiten Trainingsausgabedaten an.

In alternativer Ausführung ist die trainierte Funktion als kontinuierlich lernendes System ausgebildet, welches keine initiale Trainingsphase erfordert. Das System aktualisiert nach jeder Untersuchung das angewandte Zuordnungsmodell, welches zu Anfang überwiegend auf manuellen Eingaben des Nutzers basiert. Nach jedem Durchlauf einer Untersuchung wird der Trainingsdatensatz aktualisiert und das Zuordnungsmodell entsprechend angepasst. Diese Vorgehensweise ermöglicht jederzeit eine automatische Reaktion der trainierten Funktion auf Veränderungen in Bezug auf einrichtungsspezifische oder die Präferenzen von Nutzern. Die trainierte Funktion liefert somit ein besonders verlässliches Ergebnis. Serviceaufwände an der medizinischen Bildgebungsanlage sowie fehlerhaft bzw. unerwünschte Ermittlungen von Steuerdatensätzen reduzieren sich derart.

Insbesondere bei Inbetriebnahme eines derartigen kontinuierlich selbstlernenden Systems kann erfindungsgemäß vorgesehen sein, dass die trainierte Funktion zu definierten Indikationen lediglich eine Vorauswahl von geeigneten Steuerdaten überhaupt für eine Zuordnung in Betracht zieht, um klinisch abwegige Bildgebungsprotokolle, Rekonstruktionen und/oder Nachbearbeitungen durch theoretisch mögliche Verknüpfungen zwischen Indikationsdaten und Steuerdaten zu verhindern. Dieses a priori Wissen kann der trainierten Funktion bspw. über eine entsprechende Parametrierung bereitgestellt werden.

Anders ausgedrückt, umfasst in Ausführungen des Verfahrens das Aktualisieren der trainierten Funktion, dass mittels der Schnittstelleneinheit individuelle Zuordnungsregeln der trainierten Funktion festgelegt werden können. Insbesondere können die Zuordnungsregeln manuell durch den Nutzer festgelegt werden. Bevorzugt können die Zuordnungsregeln aber auch über ein Netzwerk automatisiert bereitgestellt werden. In Ausführungen können die Zuordnungsregeln vor einer Inbetriebnahme und/oder später während des Betriebs nachträglich festgelegt oder aktualisiert werden. Zuordnungsregeln im Sinne der Erfindung können bspw. so ausgebildet sein, dass die Menge an Protokollparametern eines Steuerdatensatzes in jedem Fall konform zu klinischen Richtlinien ist. Dies ist insbesondere dann relevant, wenn eine klinische Richtlinie aktualisiert wurde, dieses Wissen an einer medizinischen Einrichtung jedoch noch nicht angewandt wird. Die Zuordnungsregel stellt so die Einhaltung der klinischen Richtlinie in aktueller Fassung sicher. Zeigen Indikationsdaten an, dass bspw. eine Darstellung des Ca-Scorings bei der Befundung erforderlich ist, kann eine weitere Zuordnungsregel sicherstellen, dass zwingendermaßen ein Ca-Scoring Protokoll zur Bildgebung ausgeführt werden muss. Dem nicht entsprechende Protokollparameter für die Bildgebung werden so per Zuordnungsregel von vornherein ausgeschlossen.

Diese Vorgehensweise beschleunigt den Trainingsprozess, sodass besonders schnell ein zuverlässiger Betrieb der trainierten Funktion und damit eine Reduktion bzw. gar Vermeidung von Nutzerinteraktion erreicht werden kann.

In bevorzugter Ausführung des Verfahrens umfasst die trainierte Funktion ein frequenzbasiertes Modell, welches eine Häufigkeit von Werten für den wenigstens einen erfassten Präferenzparameter berücksichtigt. Das bedeutet, dass die trainierte Funktion insbesondere im Rahmen des kontinuierlichen Lernens ausgebildet ist, zu erfassen, wie oft, ein bestimmter Präferenzparameter bzw. ein Wert für einen Präferenzparameter entsprechend einer definierten Steuervorgabe für die Bildaufnahme, Rekonstruktion und/oder Bildnachbearbeitung von einem Nutzer eingegeben wurde. Die trainierte Funktion ist ferner ausgebildet, die automatisierte, modellbasierte Zuordnungsvorschrift frequenzbasiert auszuführen bzw. anzupassen.

Um dem Nutzer das Bestätigen oder eine Korrektur bzw. Anpassung eines automatisch ermittelten Steuerdatensatzes zu ermöglich, ist in weiterer Ausführung der Erfindung ein Schritt des Ausgebens des automatisch ermittelten Steuerdatensatzes mittels Schnittstelleneinheit vorgesehen. Die Ausgabe umfasst bevorzugt eine graphische Ausgabe des Steuerdatensatzes über einen Monitor der Schnittstelleneinheit. Bevorzugt umfasst die Ausgabe, jeden einzelnen Wert für jeden der vom Steuerdatensatz umfassten Protokollparameter auszugeben. Stellt der Nutzer fest, dass ein Wert für einen der Protokollparameter nicht seinen Wünschen bzw. der Handhabung der medizinischen Einrichtung entspricht, kann erfindungsgemäß vorgesehen sein, dass der Nutzer diesen Protokollparameter über die Schnittstelleneinheit abändert. Sind die Werte der Protokollparameter korrekt ermittelt worden, kann der Nutzer diese über die Schnittstelleneinheit manuell bestätigen.

Die Erfindung betrifft als weiteren Aspekt ein Computerprogrammprodukt mit einem Computerprogramm, welches direkt in eine Speichereinrichtung einer Steuereinheit einer medizinischen Bildgebungsanlage ladbar ist, mit Programmabschnitten, um einzelne oder alle Schritte des Verfahrens zur Steuerung der medizinischen Bildgebungsanlange auszuführen, wenn das Computerprogramm in der Steuereinheit ausgeführt wird.

Die Erfindung betrifft als weiteren Aspekt ferner einen Computerlesbares Medium, auf welchem von einer Rechen- oder Steuereinheit einlesbare und ausführbare Programmabschnitte gespeichert sind, um einzelne oder alle Schritte des Verfahrens zur Steuerung der medizinischen Bildgebungsanlage auszuführen, wenn die Programmabschnitte von der Recheneinheit ausgeführt werden.

Ein Computerprogrammprodukt kann Software mit einem Quellcode, der noch kompiliert und gebunden oder der nur interpretiert werden muss, oder einen ausführbaren Softwarecode umfassen, der zur Ausführung nur noch in eine Datenverarbeitungseinheit zu laden ist. Durch das Computerprogrammprodukt kann das Verfahren schnell, identisch wiederholbar und robust ausgeführt werden. Das Computerprogrammprodukt ist so konfiguriert, dass es mittels der Rechen- bzw.- Datenverarbeitungseinheit die erfindungsgemäßen Verfahrensschritte ausführen kann. Die Datenverarbeitungseinheit muss dabei jeweils die Voraussetzungen wie beispielsweise einen entsprechenden Arbeitsspeicher, einen entsprechenden Prozessor, eine entsprechende Grafikkarte oder eine entsprechende Logikeinheit aufweisen, sodass die jeweiligen Verfahrensschritte effizient ausgeführt werden können.

Das Computerprogrammprodukt ist beispielsweise auf einem computerlesbaren Medium gespeichert oder auf einem Netzwerk oder Server hinterlegt, von wo es in den Prozessor der jeweiligen Datenverarbeitungseinheit geladen werden kann. Der Server, Speicher oder das Netzwerk kann mit der Datenverarbeitungseinheit direkt verbunden oder als Teil derselben ausgebildet sein kann. Weiterhin können Steuerinformationen des Computerprogrammproduktes auf einem computerlesbaren Medium gespeichert sein. Die Steuerinformationen des computerlesbaren Mediums können derart ausgebildet sein, dass sie bei Verwendung des Datenträgers in einer Datenverarbeitungseinheit ein erfindungsgemäßes Verfahren durchführen. Beispiele für computerlesbare Speichermedien sind eine DVD, ein Magnetband oder ein USB-Stick, auf welchem elektronisch lesbare Steuerinformationen, insbesondere Software, gespeichert sind. Wenn diese Steuerinformationen von dem Datenträger gelesen und in eine Datenverarbeitungseinheit gespeichert werden, können alle erfindungsgemäßen Ausführungsformen der vorab beschriebenen Verfahren durchgeführt werden. So kann die Erfindung auch von dem besagten computerlesbaren Medium ausgehen. Die Vorteile des vorgeschlagenen Computerprogrammproduktes bzw. der zugehörigen computerlesbaren Medien entsprechen im Wesentlichen den Vorteilen der vorgeschlagenen Verfahren.

Die oben beschriebenen Eigenschaften, Merkmale und Vorteile dieser Erfindung sowie die Art und Weise, wie diese erreicht werden, werden klarer und deutlicher verständlich im Zusammenhang mit der folgenden Beschreibung der Ausführungsbeispiele, die im Zusammenhang mit den Zeichnungen näher erläutert werden. Durch diese Beschreibung erfolgt keine Beschränkung der Erfindung auf diese Ausführungsbeispiele. In verschiedenen Figuren sind gleiche Komponenten mit identischen Bezugszeichen versehen. Die Figuren sind in der Regel nicht maßstäblich. Es zeigen:

FIG 1 eine schematische Darstellung des erfindungsgemäßen Verfahrens gemäß einem Ausführungsbeispiel der vorliegenden Erfindung,

FIG 2 eine schematische Darstellung eines neuronalen Netzes zur Verwendung in einem erfindungsgemäßen Verfahrens, und

FIG 3 eine medizinische Bildgebungsanlage in Form eines Computertomographen umfassend eine erfindungsgemäße Steuereinheit gemäß einem Ausführungsbeispiel der vorliegenden Erfindung.

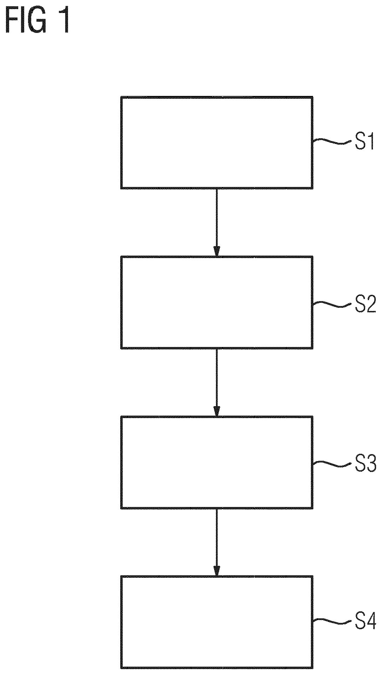

**Figur 1** zeigt ein Ablaufschema eines erfindungsgemäßen computerimplementierten Verfahrens zur Steuerung einer medizinischen Bildgebungsanlage 1.

In Schritt S1 erfolgt ein Erfassen von patientenspezifischen Indikationsdaten mittels Schnittstelleneinheit. Die Indikationsdaten können aus verschiedenen Datenquellen extrahiert, bereitgestellt bzw. abgerufen werden, bspw. können die Indikationsdaten aus einem zentralen oder dezentralen Datenspeicher, bspw. in Form einer Patienten-Datenbank abgerufen werden, bspw. aus einem RIS, HIS und/oder PACS oder einem Cloud-Speicher. Auf dem Datenspeicher kann für jeden Patienten eine elektronische Patientenakte abrufbar hinterlegt sein, sodass alle zur Verfügung stehenden elektronischen Unterlagen und Dokumente, die als Indikationsdaten dienen können, an einem (virtuellen) Ort zusammengeführt sind. Alternativ kann die elektronische Patientenakte zumindest teilweise auch auf der persönlichen Versicherten-Karte des Patienten 3 hinterlegt sein.

Die Indikationsdaten entsprechen Vorwissen über den Patienten 3. sie umfassen insbesondere Angaben über eine medizinische Bildgebungsprozedur zur Erzeugung von Bilddaten zu dem Patienten 3, um bspw. damit eine Befundung durchzuführen. Die Indikationsdaten repräsentierten insbesondere einen medizinischen bzw. anatomischen Gesamtzustand des Patienten 3. Die Indikationsdaten können in strukturierter, aber auch unstrukturierter Form vorliegen. Insofern kann Schritt S1 auch eine Strukturierung der Indikationsdaten umfassen, um bspw. freitextlich gestaltete Indikationsdate für die Steuereinheit 12 zugänglich und maschinell weiterverarbeitbar zu machen.

Schritt S1 umfasst optional das Erfassen von Zusatzinformationen über den Patienten, bspw. anatomische oder personenbezogene Informationen zu erfassen. Solche Angaben können bspw. das Alter, das Geschlecht oder Größe und Gewicht des Patient 3 betreffen. Diese Zusatzinformation kann ebenfalls in Schritt S3 berücksichtigt werden.

In Schritt S2 erfolgt ein Erfassen wenigstens eines nutzer- und/oder einrichtungsspezifischen Präferenzparameters mittels Schnittstelleneinheit. Der wenigstens eine Präferenzparameter wird folglich benutzerseitig bspw. über Anzeige- und Eingabeeinheiten 11, 7 zur Weiterverarbeitung bereitgestellt wird.

Der Präferenzparameter ist einerseits spezifisch für die erfassten Indikationsdaten zu dem zu untersuchenden Patienten. Der Präferenzparameter kennzeichnet eine bevorzugte Einstellung bzw. einen präferierten Wert für einen Parameter eines medizinischen Bildgebungsprotokolls bzw. für ein Bildrekonstruktions- oder Bildnachbearbeitungsverfahren. Der Präferenzparameter ist nutzer- und/oder einrichtungsspezifisch, er ermöglicht folglich eine Berücksichtigung von Präferenzen/Unterschieden bei der Ermittlung des zu den erfassten Indikationsdaten gehörigen Steuerdatensatzes. Der wenigstens eine Präferenzparameter ist bspw. ein Parameter kennzeichnend das Field of View für die Bilddatenerfassung, ein Kontrastmittel, welches bei der Bildgebung zum Einsatz kommt, kennzeichnend die Matrixgröße, Dosis, den Rekonstruktionskern oder die Filterung für einen Rekonstruktionsalgorithmus. Präferenzparameter für eine Bildnachbearbeitung können als Angabe ausgebildet sein, dass eine bestimmte Nachbearbeitung durchgeführt werden soll, bspw. eine virtuell native Rekonstruktion. Ein anderes Beispiel ist die Vorgabe für ein Energieniveau eines monoenergetischen Bildes, welches aus den erfassten Projektionsdaten ermittelt wird. Andere Beispiele betreffen eine Jodkarte, Rendering Parameter oder eine spezielle Segmentierung.

In Schritt S3 erfolgt ein automatisches Ermitteln eines Steuerdatensatzes basierend auf den Indikationsdaten und dem Präferenzparameter mittels Recheneinheit 21. Der Steuerdatensatz umfasst entsprechend dem wenigstens einen Präferenzparameter dabei wenigstens einen Steuerbefehl in Bezug auf eine Bilddatenerfassung mittels der medizinischen Bildgebungsanlage und/oder in Bezug auf eine Bildrekonstruktion aus den erfassten Bilddaten und/oder in Bezug auf eine Nachbearbeitung der rekonstruierten Bilddaten umfasst.

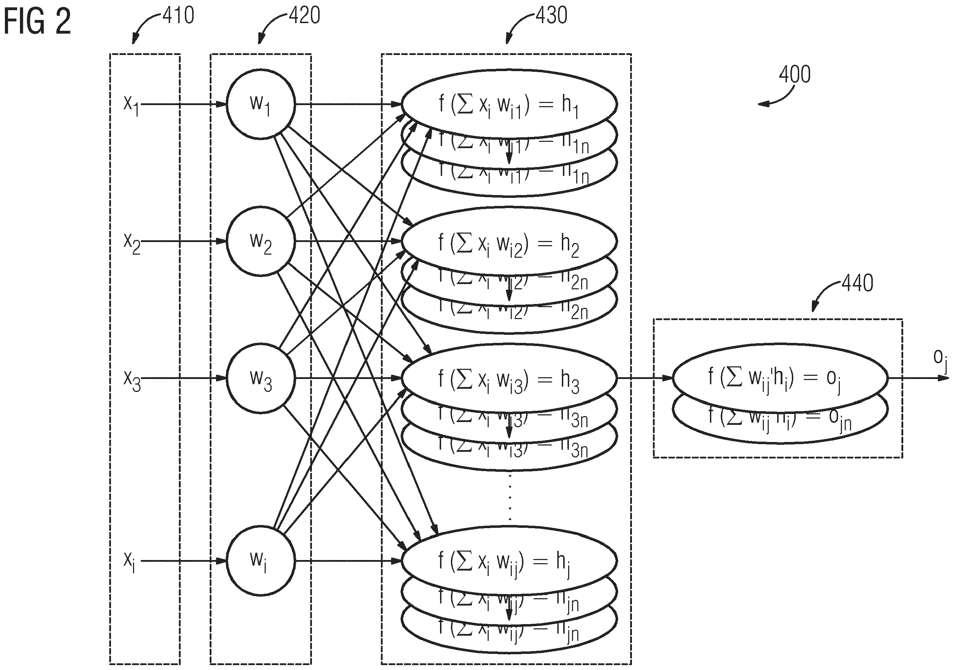

Das automatische Ermitteln des Steuerdatensatzes umfasst das Anwenden einer trainierten Funktion des maschinellen Lernens auf die Indikationsdaten. In diesem Schritt wird folglich eine Vielzahl von Steuerparametern passend zu den Indikationsdaten ermittelt, wobei die Steuerparameter als individuelle Protokollparameter zur Bilddatenerfassung, Bildrekonstruktion und/oder Bildnachbearbeitung ausgebildet sind. Die trainierte Funktion des maschinellen Lernens kann insbesondere in Form eines neuronalen Netzes 400 ausgebildet sein, wie es mit Bezug zu Figur 2 weiter unten beschrieben wird.

Schritt S3 kann in Ausführungen umfassen, dass die trainierte Funktion 400 basierend auf abgeschlossenen automatischen Ermittlungen eines Steuerdatensatzes kontinuierlich aktualisiert wird. Mit anderen Worten ist die trainierte Funktion als kontinuierlich selbstlernendes System ausgebildet. Dazu kann die Recheneinheit 21 ausgebildet sein, erfasste Präferenzparameter zu kürzlich ausgeführten Untersuchungen zu erfassen, abzuspeichern und für ein Update der Gewichte einzelner Knoten des neuronalen Netzes 400 heranzuziehen. Besonders gut kann das neuronale Netz 400 an lokale Veränderungen oder an unterschiedliche/neue Nutzer angepasst, wenn die trainierte Funktion ein frequenzbasiertes Modell umfasst, welches eine Häufigkeit von Werten für den wenigstens einen erfassten Präferenzparameters berücksichtigt. Wird per Präferenzparametereingabe für ein- und dieselben Indikationsdaten ein Wert öfter als ein anderer Wert eingegeben, so lernt das neuronale Netz über die Update-Schleife, für zukünftige Untersuchungen von vornherein dem entsprechenden Protokollparameter den ersten der beiden Werte für den Steuerdatensatz zuzuordnen. Der zweite Wert wird verworfen bzw. unterdrückt.

Insofern ist erkennbar, dass erfindungsgemäß die Schritte S1 bis S4 in einer Wiederholschleife (nicht dargestellt) für eine Vielzahl von Patienten ausgeführt werden können.

In Ausführungen kann das Aktualisieren der trainierten Funktion auch umfassen, mittels der Schnittstelleneinheit individuelle Zuordnungsregeln der trainierten Funktion 400 manuell festzulegen bzw. anzupassen. Derart wird gleichsam das neuronale Netz konfiguriert. Insbesondere werden über die Zuordnungsregeln für bestimmte Indikationsdaten, also bestimmte medizinische Konstellationen einen Patienten 3 klinisch unsinnige oder für eine Einrichtung bzw. einen Nutzer unerwünschte Steuerparameter von vornherein ausgeschlossen. Insbesondere kann über die Zuordnungsregeln die Einhaltung klinischer Richtlinien gewährleistet werden. Die Zuordnungsregeln können dazu alternativ auch über ein Netzwerk automatisiert bereitgestellt werden. Auch das Aktualisieren der trainierten Funktion 400 mittels einer Anpassung/Festlegung einer Zuordnungsregel kann in jedem Durchlauf des iterativ ausgeführten Verfahrens erfolgen. In weiterer Ausführung erfolgt das Aktualisieren bspw. immer nach fünf, acht oder zehn Durchläufen des Verfahrens.

Alternativ zu einer Ermittlung des Steuerdatensatzes mittels einer Funktion des maschinellen Lernens kann auch vorgesehen sein, dass auf an sich bekannte Weise zunächst automatisch ein Steuerdatensatz entsprechend den patientenspezifischen Indikationsdaten aus einer vorab definierten Zuordnung zwischen einer Vielzahl von verschiedenen Indikationsdaten und einer Vielzahl von verschiedenen Steuerdatensätzen vorausgewählt wird, und erst im Anschluss daran die automatische Vorauswahl des Steuerdatensatzes basierend auf dem Präferenzparameter angepasst wird. Hierbei nutzt das Verfahren zunächst etablierte Verfahren zur Zuordnung von Steuerdatensätzen zu den Indikationsdaten und modifiziert diese im Anschluss an die Nutzerpräferenzen bzw. Einrichtungsvorgaben.

In einem optionalen Schritt S5 (nicht gezeigt) kann im Rahmen des Verfahrens vorgesehen sein, den automatisch ermittelten Steuerdatensatz mittels Schnittstelleneinheit, konkret mittels Anzeigeeinheit 11 zur Bestätigung oder Anpassung durch einen Nutzer auszugeben. Für den Fall, dass das neuronale Netz trotz des kontinuierlichen Selbstlernens wenigstens einen Steuerparameter nicht nutzer- oder einrichtungskonform zu den Indikationsdaten zugewiesen hat, kann der Nutzer nun über die Eingabe eines dem Steuerparameter entsprechenden Präferenzparameter eingeben und diesen nach seinen Wünschen korrigieren, bevor der Steuerdatensatz ausgeführt wird. Alternativ kann der Nutzer die ermittelten Steuerdaten bestätigen, wenn diese wunschgemäß zugeordnet wurden. Derart realisiert das erfindungsgemäße Verfahren eine manuelle Prüfschleife.

In Schritt S4 erfolgt ein Steuern der medizinischen Bildgebungsanlage 1 mit dem ermittelten Steuerdatensatz mittels Recheneinheit 21. Mit anderen Worten wird in Schritt S4 der ermittelte Steuerdatensatz zumindest teilweise an die Aufnahmeeinheit 17 und/oder die Rekonstruktions- und Nachbearbeitungseinheit 23 gesendet und dort jeweils zur Ausführung gebracht.

Zusammenfassend werden aus vorliegenden klinischen Informationen (bspw. Krankenakte, Zuweisung, Vorbefund) zu einem Patienten 3 und möglicherweise zusätzlichen Informationen zum Patienten (= Indikationsdaten) sowie über geeignete interaktive Dialoge erfasste Nutzer- bzw. Einrichtungsvorgaben (= Präferenzparameter) automatisch Steuerdaten abgeleitet, die nicht nur spezifisch für den jeweiligen Patienten sind, sondern gleichzeitig auch nutzer- bzw. einrichtungsspezifische Präferenzen berücksichtigen. Diese Steuerdaten werden in einer Aufnahmeeinheit 17 oder einer Rekonstruktions- und Nachbearbeitungseinheit 23 der medizinischen Bildgebungsanlage 1 für eine Bilddatenakquise, Rekonstruktion oder Nachbearbeitung verwendet. Um die Indikationsdaten sowie die Präferenzparameter zu verarbeiten, werden erfindungsgemäß bevorzugt Methoden aus dem Bereich der künstlichen Intelligenz verwendet, wie neuronale Netze.

**Figur 2** zeigt ein künstliches neuronales Netz 400, wie es im Verfahren gemäß Figur 1 zum Einsatz kommen kann. Das neuronale Netz 400 antwortet auf Eingabewerte zu einer Vielzahl von Eingangsknoten $x_i$ 410 die angewendet werden, um eine oder eine Vielzahl von Ausgaben $o_j$ zu erzeugen. Das neuronale Netz 400 lernt in diesem Ausführungsbeispiel, indem es die Gewichtungsfaktoren $w_i$ (weights) der einzelnen Knoten basierend auf Trainingsdaten anpasst. Mögliche Eingabewerte der Eingangsknoten $x_i$ 410 können bspw. in Form von Indikationsdaten umfassend eine Vielzahl von Indikationsparametern, wenigstens einem oder einer Vielzahl von Präferenzparametern umfassen. Eingabewerte können weiter auch in Form von Zusatzinformation in Bezug auf den Patienten eingegeben werden, bspw. anatomische Parameter des Patienten wie bspw. Gewicht, Größe, Geschlecht oder dergleichen. Das neuronale Netz 400 ist nun ausgebildet, die Eingabewerte 410 basierend auf dem Lernprozess zu gewichten. Die Ausgabewerte 440 des neuronalen Netzes 400 entsprechen bevorzugt einer Vielzahl von Protokollparametern für eine Bilddatenerfassung, eine Bildrekonstruktion und/oder eine Bildnachbearbeitung, die als Steuerparameter in die Erstellung eines Steuerdatensatzes einfließen. Die Ausgabe 440 kann über einen einzelnen oder eine Vielzahl von Ausgabeknoten $o_j$ erfolgen. Alternativ kann das neuronale Netz ausgebildet sein, bspw. in der letzten Schicht vor der Ausgabeschicht, die mittels Lernprozess zugeordneten Protokollparameter in einer Steuerdatensatz zu überführen und diesen als Ausgabewerte 440 auszugeben.

Das künstliche neuronale Netz 400 umfasst bevorzugt eine versteckte Schicht 430, die eine Vielzahl von Knoten $h_j$ umfasst. Es können mehrere versteckte Schichten $h_{jn}$ vorgesehen sein, wobei eine versteckte Schicht 430 Ausgabewerte einer anderen versteckten Schicht 430 als Eingabewerte verwendet. Die Knoten einer versteckten Schicht 430 verrichten mathematische Operationen. Ein Ausgabewert eines Knotens $h_j$ entspricht dabei einer nicht-linearen Funktion f seiner Eingabewerte $x_i$ und der Gewichtungsfaktoren $w_i$. Nach dem Erhalt von Eingabewerten $x_i$, führt ein Knoten $h_j$ eine Summierung einer mit den Gewichtungsfaktoren $w_i$ gewichteten Multiplikation jedes Eingabewerts $x_i$ durch, wie bspw. durch folgende Funktion bestimmt:

$$h_j \,=\, f\!\left( \sum_i x_i \cdot w_{ij} \right)$$

Insbesondere wird ein Ausgabewert eines Knotens $h_j$ als Funktion f einer Knoten-Aktivierung, bspw. eine Sigmoidalfunktion oder eine lineare Rampenfunktion gebildet. Die Ausgabewerte $h_j$ werden an den bzw. die Ausgabeknoten $o_j$ übertragen. Erneut wird eine Summierung einer gewichteten Multiplikation jedes Ausgabewertes $h_j$ als Funktion der Knoten-Aktivierung f berechnet:

$$o_j \,=\, f\!\left( \sum_i h_i \cdot w'_{ij} \right)$$

Das hier gezeigte neuronale Netz 400 ist ein Feedforward neuronales Netz, bei dem alle Knoten 430 die Ausgabewerte einer vorherigen Schicht in Form ihrer gewichteten Summe als Eingabewerte verarbeiten. Selbstredend können erfindungsgemäß auch andere neuronale Netztypen zum Einsatz kommen, bspw. Feedback-Netze, bei denen ein Eingabewert eines Knotens $h_j$ gleichzeitig auch sein Ausgabewert sein kann.

Das neuronale Netz 400 wird mittels einer Methode des überwachten Lernens trainiert, um Muster zu erkennen. Eine bekannte Vorgehensweise ist die Back-Propagation, die für alle Ausführungsbeispiele der Erfindung angewandt werden kann. Während des Trainings wird das neuronale Netz 400 auf Trainings-Eingabewerten in Form einer Vielzahl von Indikationsdaten, ggf. von einer Vielzahl von Präferenzparametern und/oder von Angaben zur Nutzer-Identifikation angewandt und muss entsprechende, vorher bekannte Ausgabewerte, die TrainingsAusgabedaten in Form von einer Vielzahl von entsprechenden Steuerdatensätzen erzeugen. Iterativ werden mittlere quadratische Fehler (mean square error - "MSE") zwischen berechneten und erwarteten Ausgabewerten berechnet und einzelne Gewichtungsfaktoren 420 so lange angepasst, bis die Abweichung zwischen berechneten und erwarteten Ausgabewerten unterhalb einer vorbestimmten Schwelle liegt.

Das neuronale Netz 400 ist hier als kontinuierlich lernendes Netz ausgebildet, welches auch nach abgeschlossener Lernphase Anpassungen der erlernten Gewichtungsfaktoren $w_i$ der Vielzahl Knoten $h_j$ vornimmt, wenn sich der Zusammenhang zwischen Trainingseingabedaten und Trainingsausgabedaten mit der Zeit verändert.

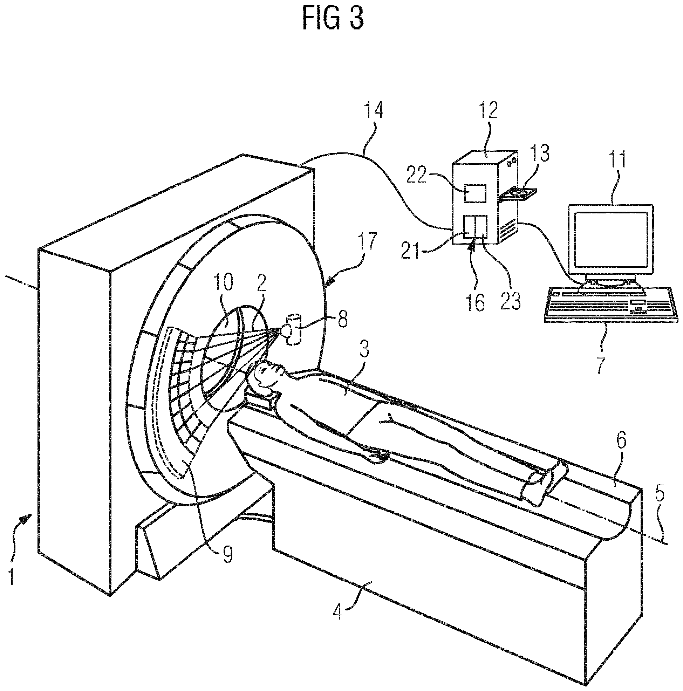

**Figur 3** zeigt eine medizinische Bildgebungsanlage 1 in Form eines Computertomographen 1. Der hier gezeigte Computertomograph 1 verfügt über eine Aufnahmeeinheit 17, umfassend eine Röntgenstrahlungsquelle 8 sowie einen Röntgenstrahlungsdetektor bzw. Röntgendetektor 9. Zur Bilddatenerzeugung rotiert die Aufnahmeeinheit 17 zur Aufnahme von Röntgenprojektionen um eine Systemachse 5, und die Röntgenquelle 8 emittiert während der Aufnahme Röntgenstrahlen 2, diese passieren den Patienten 3 und werden dabei abgeschwächt und treffen auf den Röntgendetektor 9. Welche Einstellungen bspw. an der Röntgenstrahlenquelle 8 vorgenommen werden müssen, um die gewünschten Röntgenprojektionen zu erzeugen wird mittels der Protokollparameter des gewünschten Bildgebungsprotokolls festgelegt. Protokollparameter für die Bilddatenerfassung sind bspw. die Röntgenstrahlenenergie oder FOV-Information, um den gewünschten Körperbereich des Patienten abzubilden.

Der Computertomograph 1 verfügt über eine Steuereinheit 12 in Form eines Computers umfassend eine Schnittstelleneinheit. Die Schnittstelleneinheit umfasst eine Anzeigeeinheit 11, beispielsweise zur graphischen Anzeige von medizinischen Bildaufnahmen an einem Radiologie-Arbeitsplatz der medizinischen Bildgebungsanlage 1, hier in Form von aus den Röntgenprojektionen mittels eines Rekonstruktionsverfahrens erstellten Computer-Tomographieaufnahmen oder einem Steuermenü für die Bildgebungsanlage 1. Die Schnittstelleneinheit umfasst weiter eine ebenfalls zum Radiologie-Arbeitsplatz gehörige Eingabeeinheit 7, die mit der Anzeigeeinheit 11 verbunden ist. Bei der Anzeigeeinheit 11 kann es sich beispielsweise um einen LCD-, Plasma- oder OLED-Bildschirm handeln. Es kann sich weiterhin um einen berührungsempfindlichen Bildschirm handeln, welcher auch als Eingabeeinheit 7 ausgebildet ist. Ein solcher berührungsempfindlicher Bildschirm kann in das bildgebende Gerät integriert oder als Teil eines mobilen Geräts ausgebildet sein. Bei der Eingabeeinheit 7 handelt es sich beispielsweise um eine Tastatur, eine Maus, einen sogenannten "Touch-Screen" oder auch um ein Mikrofon zur Spracheingabe. Die Eingabeeinheit 7 kann eingerichtet sein, um Bewegungen eines Nutzers zu erkennen und in entsprechende Befehle zu übersetzen. Mittels Eingabeeinheit 7, insbesondere per Sprache oder Tastatur, kann beispielsweise ein Nutzer den wenigstens einen Präferenzparameter eingeben, individuelle Zuordnungsregeln festlegen oder einen ermittelten Steuerdatensatz bzw. einzelne der darin umfassten Protokollparamater bestätigen oder anpassen.

Die Steuereinheit 12 steht mit der drehbaren Aufnahmeeinheit 17 zum Datenaustausch in Verbindung. Über die Verbindung 14 werden bspw. einerseits Steuerbefehle für die Datenakquise bzw. Bilddatenerfassung von der Steuereinheit 12 an die Aufnahmeeinheit 17 übertragen, insbesondere werden über die Verbindung 14 ermittelte Steuerparameter in Form von Protokollparametern bspw. betreffend die Röntgendosis, die Rotationsinkremente oder dergleichen übertragen. Andererseits können für den Patienten 3 aufgenommene Projektionsdaten für eine Bildrekonstruktion mittels gängiger Rekonstruktionsverfahren an den Computer 12 übertragen werden. Die Verbindung 14 ist in bekannter Weise kabelgebunden oder kabellos realisiert.

Die Steuereinheit 12 umfasst eine Datenverarbeitungseinheit 16. Sie ist eingerichtet, alle im Bezug zu dem erfindungsgemäßen Verfahren stehenden Rechenschritte durchzuführen, um basierend auf Indikationsdaten nutzer- und/oder einrichtungsspezifisch einen Steuerdatensatz zu ermitteln und die medizinische Bildgebungsanlage 1 zu steuern. Die Indikationsdaten und ggf. weitere patientenspezifische Angaben können bspw. über einen mobilen, an sich bekannten computerlesbaren Datenträger, ein Krankenhaus- oder Radiologieinformationssystem (HIS oder RIS) oder über das Internet, bspw. aus einem Cloud-Speicher oder aus einem internen Speicher auf an sich bekannte Weise über die Schnittstelleneinheit der Bilddatenverarbeitungseinheit 16 zugeführt werden.

Die Datenverarbeitungseinheit 16 umfasst Mittel für die Ausführung des erfindungsgemäßen Verfahrens, wie schon mit Bezug zu den Figuren 1 und 2 beschrieben wurde. Die Datenverarbeitungseinheit 16 umfasst folglich eine Recheneinheit 21 zum Ermitteln eines Steuerdatensatzes basierend auf den Indikationsdaten und dem wenigstens einen erfassten Präferenzparameter unter Verwendung eines trainierten Algorithmus des Maschinen-Lernens zu bestimmen. Die Recheneinheit 21 umfasst hier auch eine Zuordnungseinheit, die eingerichtet ist, vorab festgelegte bzw. durch den Nutzer jederzeit anpassbare Zuordnungsregeln zu verwalten, die dann von der Recheneinheit 21 bei der Ermittlung des Steuerdatensatzes angewandt werden. Die Zuordnungseinheit steht dazu ebenfalls über die Schnittstelleneinheit bspw. mit der Ausgabe- und Eingabeeinheit 11, 7 zum Empfang von manuellen Vorgaben, und/oder einer lokalen oder zentralen Netzeinheit in Datenaustausch, die automatisch (bspw. mittels Push-Benachrichtigung) Signale entsprechend einer Aktualisierung einer klinischen Richtlinie an die Zuordnungseinheit verschickt. Beide Einheiten können als getrennte Verarbeitungseinheiten, aber auch gemeinsam in einer Einheit ausgebildet sein. Zumindest stehen die Einheiten 21 und 23 über die Schnittstelleneinheit in Datenverbindung, um die Zuordnungsregeln der Recheneinheit 21 bereitzustellen.

Daneben umfasst die Bilddatenverarbeitungseinheit 16 auch eine Rekonstruktions- und Nachbearbeitungseinheit 23, die ausgebildet ist, Rekonstruktions- bzw. Nachbearbeitungsschritte entsprechend den von der Recheneinheit 21 erzeugten Steuerbefehlen umfassend Steuerparameter in Form von Protokollparametern für eine Rekonstruktion und/oder Nachbearbeitung auszuführen. Eine Rekonstruktion von Röntgenbildern aus den erfassten Projektionsdaten kann bspw. mittels einer gefilterten Rückprojektion erfolgen. Eine Bildnachbearbeitung umfasst typischerweise eine spezielle Bildweiterverarbeitung und/oder ein Verfahren der Bildanalyse. Bspw. kann die Bildnachbearbeitung eine dreidimensionale Darstellung der rekonstruierten Bilddaten umfassen, bspw. mittels Volume Rendering. Die Bildnachbearbeitung kann auch eine spezifische Gewebesegmentierung, bspw. für Tumorgewebe, umfassen. Entsprechend umfasst ein Steuerbefehl bspw. Steuerparameter in Form von Protokollparametern betreffend die Wahl des Filterkerns für die Rekonstruktion, die Rendering-Parameter oder die pixel-bezogenen Helligkeitsschwellwerte für die Segmentierung.

Die Einheit 23 steht über die Schnittstelleneinheit zum Empfang der Steuerbefehle betreffend eine Bildrekonstruktion oder Bildnachbearbeitung mit der Recheneinheit 21 und zur Anzeige der rekonstruierten bzw. nachbearbeiteten Bilddaten mit der Anzeigeeinheit 11 in Datenaustausch. Die Recheneinheit 21 ist ferner ebenfalls über die Schnittstelleneinheit und Verbindung 14 mit der Aufnahmeeinheit 17 verbunden, um Steuerbefehle spezielle für die Bilddatenerfassung zu übertragen.

Die Steuereinheit 12 kann insbesondere mit den genannten Komponenten bzw. Einheiten über das ‚DICOM standard interchange protocol' kommunizieren, Andere Kommunikationsprotokolle und Datenformate sind ebenfalls denkbar.

Die Steuereinheit 12 kann mit einem computerlesbaren Datenträger 13 zusammenwirken, insbesondere um durch ein Computerprogramm mit Programmcode ein erfindungsgemäßes Verfahren durchzuführen. Weiterhin kann das Computerprogramm auf dem maschinenlesbaren Datenträger abrufbar gespeichert sein. Insbesondere kann es sich bei dem maschinenlesbaren Träger um eine CD, DVD, Blu-Ray Disc, einen Memory-Stick oder eine Festplatte handeln. Die Steuereinheit 12, und damit auch ihre Sub-Komponenten, kann in Form von Hard- oder in Form von Software ausgebildet sein. Beispielsweise ist die Steuereinheit 12 als ein sogenanntes FPGA (Akronym für das englischsprachige "Field Programmable Gate Array") ausgebildet oder umfasst eine arithmetische Logikeinheit. Die Steuereinheit 12, einzelne oder alle ihrer Sub-Komponenten können alternativ dezentral angeordnet sein, z.B. können einzelne Rechenschritte des Verfahrens in einem zentralen Rechenzentrum einer medizinischen Dienstleistungseinrichtung, z.B. ein Krankenhaus, oder in der Cloud ausgeführt werden. Hierbei sind insbesondere Daten- und Patientenschutz beim Datenaustausch zu berücksichtigen. Die Steuereinheit 12 kann vollständig alternativ als Cloud-basierter Computer ausgebildet sein, wobei der Datenaustausch mit der Bildgebungsablage 1 über eine sichere Internet-Verbindung erfolgt. Die Kommunikation erfolgt in einem bevorzugten Ausführungsbeispiel per DICOM-Standard, andere Standards bzw. Datenformate sind jedoch ebenfalls möglich.

In der hier gezeigten Ausführungsform ist in einem Speicher 22 der Steuereinheit 12 wenigstens ein Computerprogramm gespeichert, welches alle Verfahrensschritte des erfindungsgemäßen Verfahrens durchführt, wenn das Computerprogramm auf dem Computer 12 ausgeführt wird. Das Computerprogramm zur Ausführung der Verfahrensschritte des erfindungsgemäßen Verfahrens umfasst Programmcode. Weiterhin kann das Computerprogramm als ausführbare Datei ausgebildet sein und/oder auf einem anderen Rechensystem als dem Computer 12 gespeichert sein. Beispielsweise kann der Computertomograph 1 so ausgelegt sein, dass der Computer 12 das Computerprogramm zum Ausführen des erfindungsgemäßen Verfahrens über ein Intranet oder über das Internet in seinen internen Arbeitsspeicher lädt.

Wo noch nicht explizit geschehen, jedoch sinnvoll und im Sinne der Erfindung, können einzelne Ausführungsbeispiele, einzelne ihrer Teilaspekte oder Merkmale miteinander kombiniert bzw. ausgetauscht werden, ohne den Rahmen der hiesigen Erfindung zu verlassen. Mit Bezug zu einem Ausführungsbeispiel beschriebene Vorteile der Erfindung treffen ohne explizite Nennung, wo übertragbar, auch auf andere Ausführungsbeispiele zu.

**Patentansprüche**

1. Computerimplementiertes Verfahren zur Steuerung einer medizinischen Bildgebungsanlage (1), umfassend die Schritte

    - Erfassen (S1) von patientenspezifischen Indikationsdaten mittels Schnittstelleneinheit,
    - Erfassen (S2) wenigstens eines nutzer- und/oder einrichtungsspezifischen Präferenzparameters mittels Schnittstelleneinheit,
- automatisches Ermitteln (S3) eines Steuerdatensatzes basierend auf den Indikationsdaten und dem Präferenzparameter mittels Recheneinheit (21), und
- Steuern (S4) der medizinischen Bildgebungsanlage mit dem ermittelten Steuerdatensatz mittels Recheneinheit (21).

2. Verfahren nach Anspruch 1, wobei das automatische Ermitteln (S3) umfasst, automatisch einen Steuerdatensatz entsprechend den patientenspezifischen Indikationsdaten aus einer vorab definierten Zuordnung zwischen einer Vielzahl von verschiedenen Indikationsdaten und einer Vielzahl von verschiedenen Steuerdatensätzen auszuwählen.

3. Verfahren nach Anspruch 2, wobei das automatische Ermitteln (S3) umfasst, die automatische Auswahl eines Steuerdatensatzes basierend auf dem Präferenzparameter anzupassen.

4. Verfahren nach einem der vorherigen Ansprüche, wobei das automatische Ermitteln (S3) des Steuerdatensatzes umfasst, eine trainierte Funktion (400) des maschinellen Lernens auf die Indikationsdaten anzuwenden.

5. Verfahren nach Anspruch 4, wobei die trainierte Funktion (400) als eine Funktion ausgebildet ist, die mit Trainingsdaten umfassend Trainingseingabedaten in Form einer Vielzahl von Indikationsdaten und einer Vielzahl von Präferenzparametern und Trainingsausgabedaten in Form einer Vielzahl von entsprechenden Steuerdatensätzen trainiert wurde.

6. Verfahren nach einem der Ansprüche 4 oder 5, wobei die trainierte Funktion (400) basierend auf abgeschlossenen automatischen Ermittlungen eines Steuerdatensatzes kontinuierlich aktualisiert wird.

7. Verfahren nach Anspruch 6, wobei die trainierte Funktion ein frequenzbasiertes Modell umfasst, welches eine Häufigkeit von Werten für den wenigstens einen erfassten Präferenzparameter berücksichtigt.

8. Verfahren nach einem der Ansprüche 6 oder 7, wobei das Aktualisieren der trainierten Funktion umfasst, mittels Schnittstelleneinheit individuelle Zuordnungsregeln der trainierten Funktion manuell festzulegen.

9. Verfahren nach einem der vorherigen Ansprüche, ferner umfassend dem Schritt

   - Ausgeben eines automatisch ermittelten Steuerdatensatzes mittels Schnittstelleneinheit zur Bestätigung oder Anpassung durch einen Nutzer.

10. Verfahren nach einem der vorherigen Ansprüche, wobei der Steuerdatensatz wenigstens einen Steuerbefehl in Bezug auf eine Bilddatenerfassung mittels der medizinischen Bildgebungsanlage (1) und/oder in Bezug auf eine Bildrekonstruktion aus den erfassten Bilddaten und/oder in Bezug auf eine Nachbearbeitung der rekonstruierten Bilddaten umfasst.

11. Verfahren nach einem der vorherigen Ansprüche, wobei der wenigstens eine Präferenzparameter einen Parameter für die Bilddatenerfassung mittels der medizinischen Bildgebungsanlage und/oder für eine Bildrekonstruktion aus den erfassten Bilddaten und/oder für eine Nachbearbeitung der rekonstruierten Bilddaten umfasst.

12. Steuereinheit (12) zur Steuerung einer medizinischen Bildgebungsanlage (1), umfassend

   - eine Schnittstelleneinheit (11, 7), die ausgebildet ist zum
   - Erfassen von patientenspezifischen Indikationsdaten, und

   - Erfassen wenigstens eines nutzer- und/oder einrichtungsspezifischen Präferenzparameters,
   - eine Recheneinheit (21), die ausgebildet ist zum
   - automatischen Ermitteln eines Steuerdatensatzes basierend auf den Indikationsdaten und dem Präferenzparameter, und
   - Steuern der medizinischen Bildgebungsanlage (1) mit dem ermittelten Steuerdatensatz.

13. Medizinische Bildgebungsanlage (1) umfassend eine Steuereinheit (12) nach Anspruch 12.

14. Computerprogrammprodukt mit einem Computerprogramm, welches direkt in eine Speichereinrichtung (22) einer Steuereinheit (12) einer medizinischen Bildgebungsanlage (1) ladbar ist, mit Programmabschnitten, um Schritte des Verfahrens nach einem der Ansprüche 1 bis 11 auszuführen, wenn das Computerprogramm in der Steuereinheit (12) ausgeführt wird.

15. Computerlesbares Medium (13), auf welchem von einer Recheneinheit (21) einlesbare und ausführbare Programmabschnitte gespeichert sind, um Schritte des Verfahrens nach einem der Ansprüche 1 bis 11 auszuführen, wenn die Programmabschnitte von der Recheneinheit (21) ausgeführt werden.

FIG 1

```
┌──────────────┐
│              │── S1
│              │
└──────┬───────┘
       │
       ▼
┌──────────────┐
│              │── S2
│              │
└──────┬───────┘
       │
       ▼
┌──────────────┐
│              │── S3
│              │
└──────┬───────┘
       │
       ▼
┌──────────────┐
│              │── S4
│              │
└──────────────┘
```

FIG 2

EP 4 345 835 A1

# FIG 3

**Europäisches Patentamt**
**European Patent Office**
**Office européen des brevets**

# EUROPÄISCHER RECHERCHENBERICHT

**Nummer der Anmeldung**

**EP 22 19 8687**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2018/144243 A1 (HSIEH JIANG [US] ET AL) 24. Mai 2018 (2018-05-24) <br> * Absatz [0136] * <br> * Absatz [0146] * <br> * Absatz [0152] * <br> * Absatz [0178] * <br> * Absatz [0194] – Absatz [0195] * <br> * Absatz [0203] * <br> * Absatz [0210] – Absatz [0211] * <br> * Absatz [0225] * <br> * Abbildungen 15A, 19, 21 * <br> ----- | 1-15 | INV. <br> G16H30/40 <br> G16H30/20 <br> A61B5/05 <br> A61B6/00 <br><br> ADD. <br> G06N3/08 <br> G06N3/02 |
| X | US 2019/139271 A1 (TUNG CHI-HUA [US] ET AL) 9. Mai 2019 (2019-05-09) <br> * Absatz [0022] – Absatz [0041] * <br> * Ansprüche 5, 8 * <br> ----- | 1-15 | |
| X | US 2021/183055 A1 (RAO GIREESHA CHINTHAMANI [US] ET AL) 17. Juni 2021 (2021-06-17) <br> * Absatz [0026] – Absatz [0056] * <br> * Abbildungen 2, 3 * <br> * Ansprüche 1, 2, 4 * <br> ----- | 1-15 | |

**RECHERCHIERTE SACHGEBIETE (IPC)**

A61B
G16H

Der vorliegende Recherchenbericht wurde für alle Patentansprüche erstellt

| Recherchenort | Abschlußdatum der Recherche | Prüfer |
|---|---|---|
| München | 16. Februar 2023 | Hauber, Jörg |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 22 19 8687

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-02-2023

| Im Recherchenbericht angeführtes Patentdokument | Datum der Veröffentlichung | Mitglied(er) der Patentfamilie | Datum der Veröffentlichung |
|---|---|---|---|
| US 2018144243 A1 | 24-05-2018 | KEINE | |
| US 2019139271 A1 | 09-05-2019 | CN 106537398 A | 22-03-2017 |
| | | CN 114530234 A | 24-05-2022 |
| | | EP 3170112 A1 | 24-05-2017 |
| | | US 2017206680 A1 | 20-07-2017 |
| | | US 2019139271 A1 | 09-05-2019 |
| | | WO 2016009309 A1 | 21-01-2016 |
| US 2021183055 A1 | 17-06-2021 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 3451211 A1